# EUROPEAN PATENT SPECIFICATION

(11) **EP 2 398 505 B1**
(45) Date of publication and mention of the grant of the patent: **18.06.2014**
(21) Application number: 09830706.9
(22) Date of filing: 30.11.2009
(51) Int. Cl.: A61L 2/07, B02C 19/00, B09B 3/00

(54) **WASTE TREATMENT AUTOCLAVE TO PROVIDE FOR STEAM-ASSISTED DRYING**
AUTOKLAV ZUR BEHANDLUNG VON ABFALL FÜR DAMPFGESTÜTZTE TROCKNUNG
AUTOCLAVE DE TRAITEMENT DES DÉCHETS POUR ASSURER UN SÉCHAGE À LA VAPEUR

(30) Priority: 01.12.2008 US 315258
(43) Date of publication of application: 28.12.2011
(73) Proprietor: Estech, LLC, Canal Winchester, OH 43110 (US)
(72) Inventor: MICHALEK, Jan, K., Pataskala OH 43062 (US); THOMAS, Theodore, J., Columbus OH 43235 (US)
(74) Representative: Howe, Steven
(86) International application number: PCT/US2009/006310
(87) International publication number: WO 2010/065088

(56) References cited:
- WO-A2-2006/056768
- WO-A2-2006/121697
- WO-A2-2008/112168
- DE-A1- 2 550 142
- GB-A- 2 370 242
- US-A1- 2008 217 444
- US-A1- 2008 230 484
- US-B1- 6 752 956

## Description

### Technical Field

The present invention relates to the treatment of municipal solid waste and the like. This invention disclosure presents a means of utilizing steam to heat and dry wet heterogeneous solids inside a rotating autoclave with at least one entry and exit door.

### Background of the Invention

The present invention relates to systems and methods for treating process material and, more particularly, to systems and methods for treating municipal solid waste material, medical waste material, reclaimed paper and the like.

This process typically involves sterilizing high density materials such as glass, plastics, metals and recovering others from municipal solid waste (MSW) and converting paper, cardboard, food waste, etc. to a usable fiber and separating it from other recyclable materials.

As a result of increasing scarcity of landfills and more stringent environmental regulations, efforts have been made to reduce the volume of process material, such as municipal solid waste (MSW) and paper material, such as newsprint and other reclaimed and recycled paper products as a step in the process of disposing of the material, either by depositing it in landfills, incinerating it or recycling it.

Systems and methods have been developed to break down such material for disposal, or in the case of paper products, use as insulation, or for further processing to produce a combustible product.

It remains desirable to be able to efficiently dry a treated waste mass within an autoclave as it is beneficial to render the waste mass as a relatively dry mass for further processing, such as into fuel pellets, or as fuel for use in fluidized bed combustors and the like.

One of the disadvantages of the arrangements of the prior art is that many autoclaves feature helical blades that resist material flow when the waste is loaded into the autoclave and when the treated waste mass is removed from the autoclave. Helical blades also create blockages when stringy, fibrous material, such as clothing and rugs, is present.

Another disadvantage of prior systems is that they have not been able to provide efficient in situ drying, and to make efficient and effective use of waste steam for such a process.

In this same regard, the environment surrounding the autoclave upon loading differs from that surrounding the autoclave upon discharge. As these environments differ, it is also desirable to be able to provide a waste treatment autoclave that may be loaded, operated or discharged while maintaining some degree of separation or isolation of the loading environment from the discharge environment.

The present invention accordingly represents an improvement over prior art apparatus and methods, such as those described in U.S. Patents Nos. 5,540,391; 5,126,363; 5,253,764; 5,190,226; 5,361,994; 5,427,650; 5,407,809; 5,636,449; 5,655,718 and 6,397,492, PCT application PCT/US06/16773 and PCT/US 2008/11 2168.

Recent examples (see, for example, patent 5,636,449) provide technology improvements to systems that provide steam for the use of a pipe or pipes situated longitudinally in a configuration known as a tube dryer. Steam, introduced to these tubes, heats the autoclave contents directly, evaporating contained water.

Turbulence introduced by rotating assures that the entire contents (of homogenous wetcake) are properly heated and dried.

In tube dryers, steam is introduced into, and water/steam is exhausted through, a common rotating union on one end of the autoclave. The end of the autoclave provides manifolds to distribute the steam/collect the spent steam to and from the tubes in the autoclave.

The prior art provides for the re-use of spent contact steam via "regeneration." The spent steam is separated from liquid condensate and is combined with superheated and energetic steam in a thermal vapor recovery (TVR) device, produces a product saturated steam with elevated temperature and pressure for re-use in the dryer. Contact condensate is recirculated to the boiler, and a small (5%) bleed of steam from the TVR removes non-condensibles (with a significant loss of energy). Non-contact condensate is recirculated to the boiler.

Experience with waste autoclaves shows that the contact steam has contaminants such as organic vapors. The current art eventually results in the detrimental release of the vapors to the contact steam condensate. Further, experience shows that spent contact steam has substantial air content, with detrimental consequences to both heat transfer coefficient and to tube/boiler/plumbing material life.

### Summary of the Invention

The invention includes apparatus and methods for treating solid waste using an autoclave, and especially for drying waste and recycling the steam from the autoclave system.

### Apparatus with Straight Hollow Blades for Steam Conduction

The invention includes an apparatus for processing solid waste products, said apparatus comprising: (a) a rotatably mounted cylindrical autoclave having an interior surface and two ends, at least one end terminating in a hatch that may be opened to allow access to the interior of said autoclave and sealably closed to allow pressurization of said autoclave; (b) an autoclave steam inlet for injecting contact steam into said autoclave so as to come into contact with waste material placed into said autoclave; (c) a plurality of straight hollow blades adapted to conduct non-contact steam and protruding from said interior surface of said autoclave; and (d) at least one blade steam inlet for injecting steam into said blades, in which the blades extend from one end of the autoclave to the other.

It is preferred that the autoclave additionally comprises a waste steam outlet adapted to conduct waste contact steam from said interior of said autoclave and wherein another waste steam outlet is adapted to conduct said non-contact waste steam from said blades.

It is also preferred that the autoclave steam inlet and the blade steam inlet are each provided with a rotating connector so as to permit the supply of steam therethrough while the autoclave is rotated. Typically and preferably, the steam introduced into the blades at least partially comprises steam reconstituted from non-contact blade condensate obtained from the autoclave.

A variable flow valve may be used to dynamically control the exit steam quality.

The invention also preferably features the autoclave additionally comprising a waste steam outlet adapted to conduct waste steam from the interior of the autoclave and wherein another waste steam outlet is adapted to conduct non-contact waste steam from the hollow blades; and a device that is adapted to reconstitute the waste steam from the hollow blades and to reintroduce reconstituted steam into the autoclave steam inlet and or the inlets of other autoclaves. This embodiment may additionally comprise a steam separator followed by mechanical vapor recompression device for separated steam and a boiler/steam generator for separated water.

### Method of Treating Waste Involving Steam Heating Using Contact and Non-Contact Steam

The present invention also includes a method of processing solid waste material in an autoclave, comprising: (1) loading the autoclave with a charge of solid waste products, the autoclave comprising: (a) a rotatably mounted cylindrical autoclave having an interior surface and two ends, at least one end terminating in a hatch that may be opened to allow access to the interior of the autoclave and sealably closed to allow pressurization of the autoclave; (b) an autoclave steam inlet for injecting steam into the autoclave so as to come into contact with waste material placed into the autoclave; (c) a plurality of straight hollow blades adapted to conduct steam and protruding from the interior surface of the autoclave; and (d) at least one blade steam inlet for injecting steam into the blades; (2) sealing the autoclave; (3) rotating the autoclave so as to cause the charge of solid waste material to be moved from the bottom of the autoclave to toward the top of the autoclave while introducing steam into the autoclave and into the blades, so as to produce dried processed waste; and (4) thereafter depressurizing the autoclave and unloading the processed waste therefrom.

It is preferred that the steam introduced into the blades is substantially comprised of reconstituted non-contact waste steam obtained from the autoclave, and most preferably that a variable flow valve controls the flow of the waste steam.

It is also preferred that the device adapted to reconstitute the non-contact waste steam is a steam separator followed by mechanical vapor recompression device for separated steam and a boiler/steam generator for separated water, wherein the thermal energy of the contact condensate is recovered by heat transfer to combustion air, to steam generation, and/or by direct mechanical vapor recompression.

### Method of Treating Waste Involving Steam Heating Musing Contact Steam for Processing and Non-Contact Steam for Drying

The methods of the present invention also include a method of processing solid waste material in an autoclave, comprising: (1) loading the autoclave with a charge of solid waste products, the autoclave comprising: (a) a rotatably mounted cylindrical autoclave having an interior surface and two ends, at least one end terminating in a hatch that may be opened to allow access to the interior of the autoclave and sealably closed to allow pressurization of the autoclave; (b) an autoclave steam inlet for injecting steam into the autoclave so as to come into contact with waste material placed into the autoclave; (c) a plurality of hollow blades adapted to conduct steam and protruding from the interior surface of the autoclave; (d) at least one blade steam inlet for injecting steam into the blades; and (e) a waste steam outlet adapted to conduct waste steam from the interior of the autoclave and wherein another waste steam outlet is adapted to conduct non-contact waste steam from the blades, (2) sealing the autoclave; (3) rotating the autoclave so as to cause the charge of solid waste material to be moved from the bottom of the autoclave to toward the top of the autoclave while introducing steam into the autoclave, so as to produce processed waste; (4) thereafter depressurizing the autoclave; (5) introducing waste steam into the blades so as to dry the processed waste; and (6) unloading the processed waste from the autoclave.

The present invention also includes a variation of an autoclave for steam treatment and drying comprising: a) a rotating steam tube dryer comprising a plurality of tubes, a rotating input port adapted to supply steam to the plurality of tubes, a rotating output port adapted to accept steam to the plurality of tubes and one or more end doors; b) a source of saturated or supersaturated steam connected to the steam tube dryer; c) a conduit for connecting steam through the rotating input port to the plurality of tubes; d) a conduit for withdrawing condensed steam from the plurality of tubes through the rotating output port; e) a conduit for withdrawing product moisture through a rotating port; f) a valve for controlling product moisture withdrawal through an external and non-rotating valve.

It is preferred that the spent non-contact steam and condensate are recycled back to the steam source.

The present invention also includes a method of modifying the autoclave tilt angle to a slightly off level, so as the liquid condensate within the blades is caused to migrate from the steam supply end to the steam exhaust end, the means provided by leveling actuators as described herein. This may be done for instance by a means for controlling the autoclave tilt that senses the blade steam exhaust condensate conditions and adjusting the tilt to properly regulate the production and exhaust of condensate in the blades.

The present invention represents an improvement upon these systems via a system that heats contents with both contact and non-contact steam (maintaining separation of the contact steam from the non-contact steam) by distributing the non-contact steam for drying of contents via a system of blades that serve to both heat the contents and to mechanically agitate the contents, by maintaining the pressure of the steam in the blades so as to transfer heat released by the condensation of steam in the blades, and by an efficient system of regeneration of non-contact condensate that includes a mechanical vapor recompressor.

Condensate leaving the autoclave consists of contact steam that has been in direct contact with the waste contents. Contact steam has small quantities of particulate, air, and organic vapors. While prior art provides for the removal of non-condensibles by purging a small quantity of steam, such a process loses the energy of the steam. The present invention provides for the condensation of the contact steam (with functional use for the heat of condensation), thus providing for the separation of non-condensibles, and the subsequent purification of the water column.

It is preferred that the steam condensate from the vanes is under medium pressure (typically about 5 bar to about 7 bar), but lower than supply steam pressure (typically about 7 bar to about 8 bar). Non-contact steam condensate from the autoclave is at nearly the supply steam pressure. A standard single stage mechanical vapor recompressor (MVR) is able to lift the steam to supply pressure. The present invention features the inclusion and use of such an MVR in its preferred embodiment, but allows thermal vapor recompression as an alternate.

Various designs, mechanical arrangements and technologies exist to transfer steam across the door of the autoclave and to maintain the non-contact steam.

The doors provide for dual unions, to transfer steam and condensate from fixed locations to the rotating autoclave. A system of transferring steam from the autoclave door (removable) to the autoclave proper is also provided, using an annular groove on both sides of the door seal. This allows the door to be removed and returned without an alignment problem.

In general terms, the invention includes an apparatus, system, waste treatment facility and method of autoclave operation and waste treatment.

The present invention also includes a method of changing the autoclave tilt angle to a slightly off level, such that the liquid condensate within the blades is caused to migrate from the steam supply end to the steam exhaust end. This may be done by any mechanical means, such as through the use of actuators such as those shown in Figure 1 and which are described in U.S. Patent No. 7,347,391. The present invention also features a means of controlling the autoclave tilt in by sensing thj blade steam exhaust condensate conditions and adjusting the tilt to properly regulate the production and exhaust of condensate in the blades. This may be done for instance by using temperature and pressure sensors with functional algorithmic controls that provide feedback to the tilt actuators.

### Apparatus with Straight Hollow Blades for Steam Conduction

The present invention includes an apparatus for processing solid waste products, the apparatus comprising: (a) a rotatably mounted cylindrical autoclave having an interior surface and two ends, at least one end terminating in a hatch that may be opened to allow access to the interior of the autoclave and sealably closed to allow pressurization of the autoclave; (b) an autoclave steam inlet for injecting steam into the autoclave so as to come into contact with waste material placed into the autoclave; (c) a plurality of hollow blades adapted to conduct steam and protruding from the interior surface of the autoclave; and (d) at least one blade steam inlet for injecting steam into the blades.

The apparatus may feature an autoclave that additionally comprises a waste steam outlet adapted to conduct waste steam from the interior of the autoclave and wherein the at least one blade steam inlet is connected to the waste steam outlet.

It is also preferred that the autoclave steam inlet and the blade steam inlet are each provided with a rotating connector so as to permit the supply of steam therethrough while the autoclave is rotated.

The autoclave may also optionally comprise a waste steam outlet adapted to conduct waste steam (contact steam) from the interior of the autoclave and wherein the at least one blade steam outlet is separately provided to conduct non-contact condensate from the blade or blades.

Also preferred, where the steam is introduced into the blades, is the inclusion of a variable flow valve adapted to control the non-contact condensate flow.

In a preferred variation the autoclave additionally comprises (1) a waste steam outlet adapted to conduct waste steam from the interior of the autoclave (2) at least one blade steam outlet for conducting waste steam from the hollow blades, and (3) a device adapted to reconstitute the waste steam from the hollow blades and to reintroduce reconstituted steam into the autoclave steam inlet. The device adapted to reconstitute the waste steam may be any suitable device, such as a mechanical vapor recompression device.

It is preferred that the blades are substantially straight.

### Method of Treating Waste Involving Steam Heating Using Contact and Non-Contact Steam

The invention also includes methods that may be carried out by the device.

These methods includes a method of processing solid waste material in an autoclave, comprising: (1) loading the autoclave with a charge of solid waste products, the autoclave comprising: (a) a rotatably mounted cylindrical autoclave having an interior surface and two ends, at least one end terminating in a hatch that may be opened to allow access to the interior of the autoclave and sealably closed to allow pressurization of the autoclave; (b) an autoclave steam inlet for injecting steam into the autoclave so as to come into contact with waste material placed into the autoclave; (c) a plurality of hollow blades adapted to conduct steam and protruding from the interior surface of the autoclave; and (d) at least one blade steam inlet for injecting steam into the blades; (2) sealing the autoclave; (3) rotating the autoclave so as to cause the charge of solid waste material to be moved from the bottom of the autoclave to toward the top of the autoclave while introducing steam into the autoclave and into the blades, so as to produce processed waste; and (4) thereafter depressurizing the autoclave and unloading the processed waste therefrom.

It is preferred that the method also involve use of an apparatus as is described above in its preferred and optional variations, and that the method include the additional process steps brought about by those preferred and optional elements and devices.

### Method of Treating Waste Involving Steam Heating Using Contact Steam for Processing and Non-Contact Steam for Drying

The present invention also includes a method of processing solid waste material in an autoclave, comprising: (1) loading the autoclave with a charge of solid waste products, the autoclave comprising: (a) a rotatably mounted cylindrical autoclave having an interior surface and two ends, at least one end terminating in a hatch that may be opened to allow access to the interior of the autoclave and sealably closed to allow pressurization of the autoclave; (b) an autoclave steam inlet for injecting steam into the autoclave so as to come into contact with waste material placed into the autoclave; (c) a plurality of hollow blades adapted to conduct steam and protruding from the interior surface of the autoclave; (d) at least one blade steam inlet for injecting non-contact steam into the blades; and (e) a waste steam outlet adapted to conduct contact waste steam from the interior of the autoclave and wherein the at least one blade steam inlet is connected to the waste steam outlet; (2) sealing the autoclave; (3) rotating the autoclave so as to cause the charge of solid waste material to be moved from the bottom of the autoclave to toward the top of the autoclave while introducing steam into the autoclave, so as to produce processed waste; (4) thereafter depressurizing the autoclave; (5) introducing waste steam into the blades so as to dry the processed waste; and (6) unloading the processed waste from the autoclave.

The apparatus and method of the present invention may also feature the use of steam introduced into the blades that is comprised of reconstituted non-contacted waste steam obtained from the autoclave.

The apparatus and method of the present invention may also involve the waste steam being controlled by a variable flow valve.

The apparatus of the present invention may also incorporate a device adapted to reconstitute the non-contact waste steam which is a steam separator followed by mechanical vapor recompression device for separated steam and a boiler/steam generator for separated water, such that the thermal energy of the contact condensate is recovered by heat transfer to combustion air, to steam generation, and/or by direct mechanical vapor recompression.

It is preferred that the method also involve use of an apparatus as is described above in its preferred and optional variations, and that the method include the additional process steps brought about by those preferred and optional elements and devices. It is preferred that the steam introduced into the blades is predominately regenerated condensate, and that the flow of the waste steam is reduced by a controllable variable flow valve during the drying step.

It is also preferred that the steam exiting the blades is reconstituted and reintroduced in the autoclave steam inlet.

The present invention also involves variations of the apparatus and method including the use of method of modifying the autoclave tilt angle to a slightly off level, so as the liquid condensate within the blades is caused to migrate from the steam supply end to the steam exhaust end, such as by leveling actuators as described herein and in the incorporated references.

The present invention also involves variations of the apparatus and method including means of controlling the autoclave tilt by sensing the blade steam exhaust condensate conditions (such as temperature) and adjusting the tilt to properly regulate the production and exhaust of condensate in the blades.

Other embodiments of the present invention include an autoclave for steam treatment and drying comprising: (a) a rotating steam tube dryer comprising a plurality of tubes, a rotating input port adapted to supply steam to the plurality of tubes, a rotating output port adapted to accept steam to the plurality of tubes and one or more end doors; (b) a source of saturated or supersaturated steam connected to the steam tube dryer; (c) a conduit or other means for connecting steam through the rotating input port to the plurality of tubes; (d) a conduit or other means for withdrawing condensed steam from the plurality of tubes through the rotating output port; (e) a conduit or other means for withdrawing product moisture through a rotating port; (f) a valve or other means for of controlling product moisture withdrawal through an external and non-rotating valve.

It is preferred that the spent waste non-contact steam and condensate are condensed and cleansed prior to being recycled back to the steam generator.

The invention also includes a steam cooking and drying apparatus comprising: (a) a rotating steam tube dryer comprising a plurality of tubes and one or more end doors; (b) a source of saturated or supersaturated steam connected to the dryer; (c) a conduit or other means of connecting steam through a rotating port to the plurality of tubes; (d) a conduit or other means of connecting the plurality of tubes to a discharge port, preferably comprising (1) a steam/water separator and (2) a rotating valve; and (e) a conduit or other means of withdrawing spent steam and product moisture through a rotating valve.

The autoclave contents typically are materials to be sterilized and dried, and preferably pulped and dried, such as municipal wastes.

It is preferred that, as the processing of the waste mass proceeds into the operational cycle, the flow of steam through the blades is slowed so as to compensate for the reduced rate of heat transfer as the waste mass dries. For instance, where a treatment cycle is approximately 1 hour from loading to unloading, the reduction in the steam flow rate will begin about 20 minutes from the end of the cycle. The rate of slowing will depend upon the heat transfer rate experienced with different types of waste, having varying in-coming moisture amounts, the amount of water brought in through steam contact during processing and the ambient temperature and pressure.

### Brief Description of the Drawings

Figure 1 is a perspective view of an autoclave apparatus for processing solid waste products, in accordance with one embodiment of the present invention.
Figure 2 is a perspective view of an autoclave vessel for processing solid waste products, in accordance with one embodiment of the present invention.
Figure 3 is partially sectioned perspective view of an autoclave vessel for processing solid waste products, in accordance with one embodiment of the present invention.
Figure 4 is partially sectioned perspective view of an autoclave vessel for processing solid waste products, in accordance with one embodiment of the present invention.
Figure 5 is an elevation view of an autoclave vessel for processing solid waste products, in accordance with one embodiment of the present invention.
Figure 5a is a longitudinal sectioned view, taken along line C-C of Figure 5, of an autoclave vessel for processing solid waste products, in accordance with one embodiment of the present invention.
Figure 5b is a longitudinal sectioned view, taken along line E-E of Figure 5, of an autoclave vessel for processing solid waste products, in accordance with one embodiment of the present invention.
Figure 6 is a longitudinal view of an autoclave vessel for processing solid waste products, in accordance with one embodiment of the present invention.
Figure 6a is a sectioned elevation view, taken along line G-G of Figure 6, of an autoclave vessel for processing solid waste products, in accordance with one embodiment of the present invention.
Figure 7 is a longitudinal view of an autoclave vessel for processing solid waste products, in accordance with one embodiment of the present invention.
Figure 7a is a sectioned elevation view, taken along line J-J of Figure 7, of an autoclave vessel for processing solid waste products, in accordance with one embodiment of the present invention.
Figure 7b is a detailed sectioned elevation view, taken along line L-L of Figure 7, of an autoclave vessel for processing solid waste products, in accordance with one embodiment of the present invention.
Figure 8 is sectioned perspective view of an autoclave vessel for processing solid waste products, in accordance with one embodiment of the present invention.

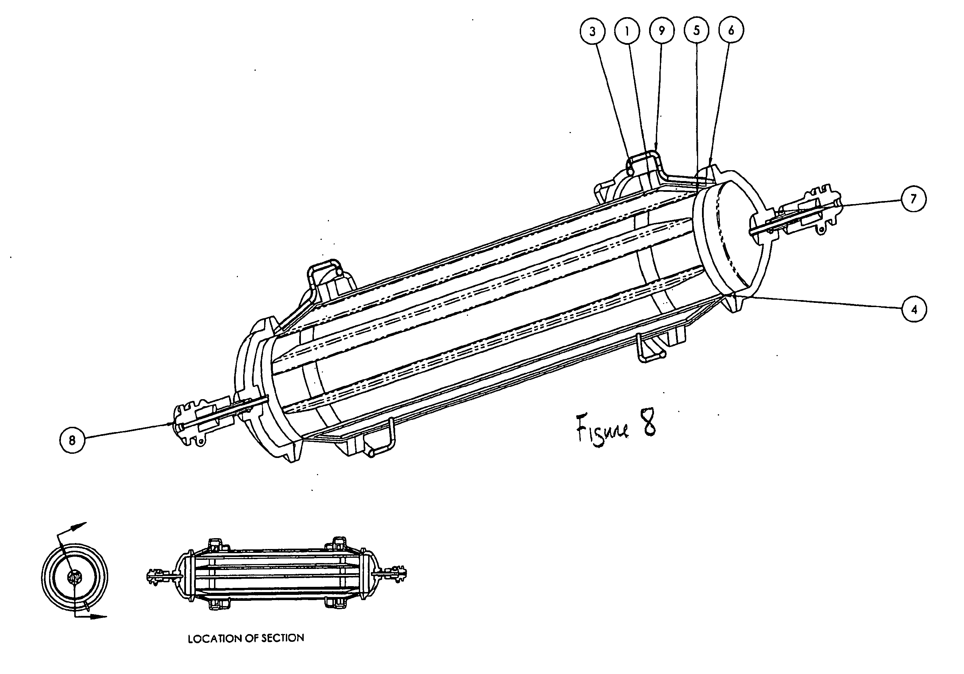

Figure 8 is schematic of a system for processing solid waste products, in accordance with one embodiment of the present invention.

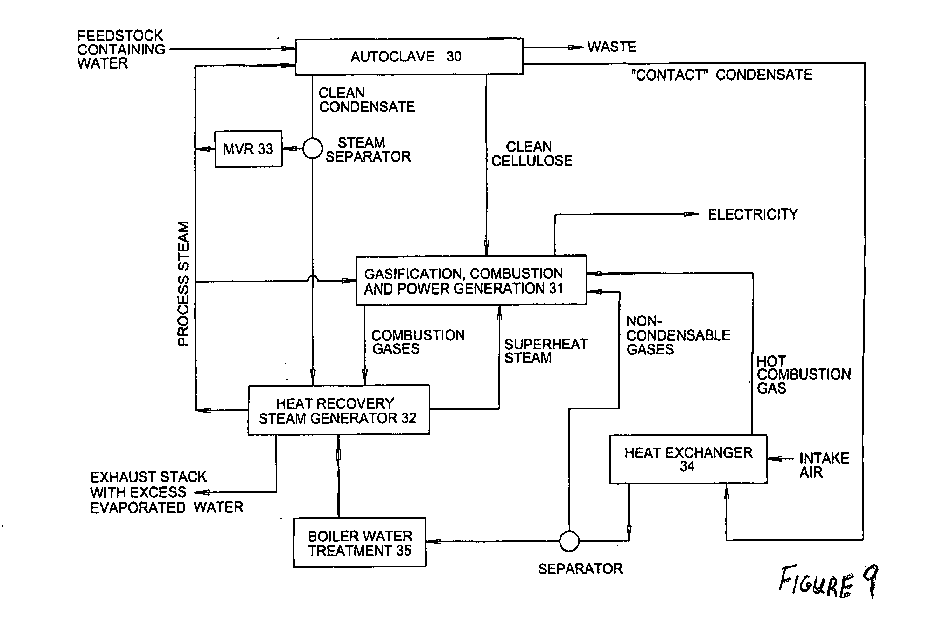

Figure 9 is a schematic for the process (steam) flow, detailing the contact steam and the autoclave, in accordance with one embodiment of the present invention.

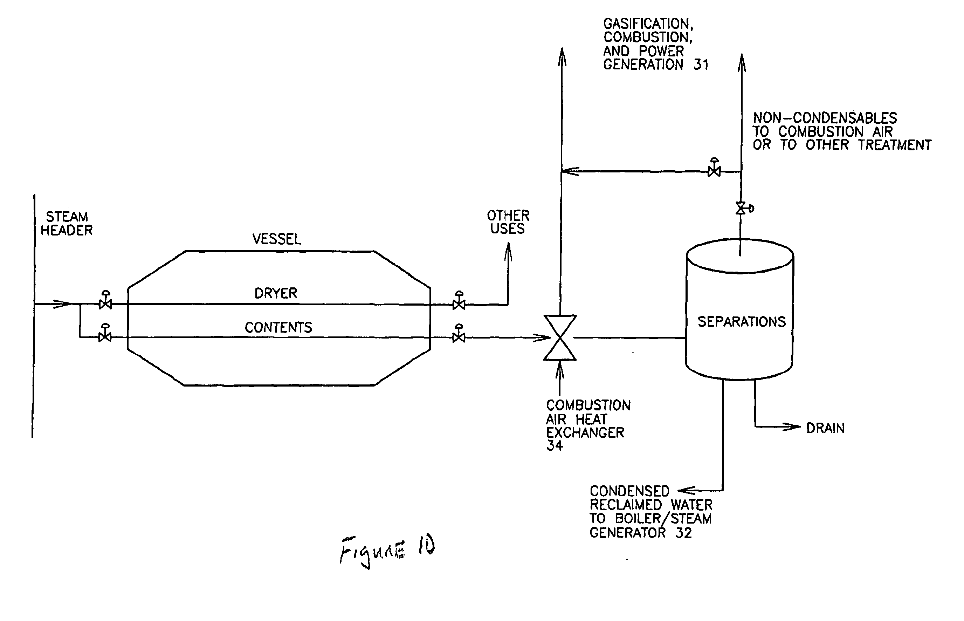

Figure 10 is a general schematic for the preferred embodiment of the autoclave contact steam recycling system, integrated with other functional elements of the preferred embodiment of the total autoclave system, in accordance with one embodiment of the present invention.

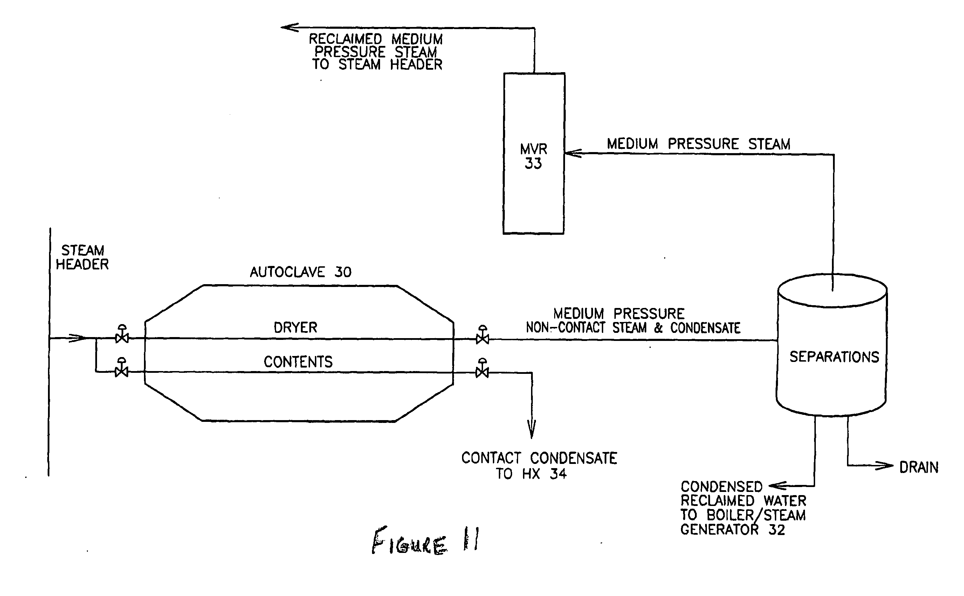

Figure 11 is a schematic for the process (steam) flow, detailing the non-contact steam and the autoclave, in accordance with one embodiment of the present invention.

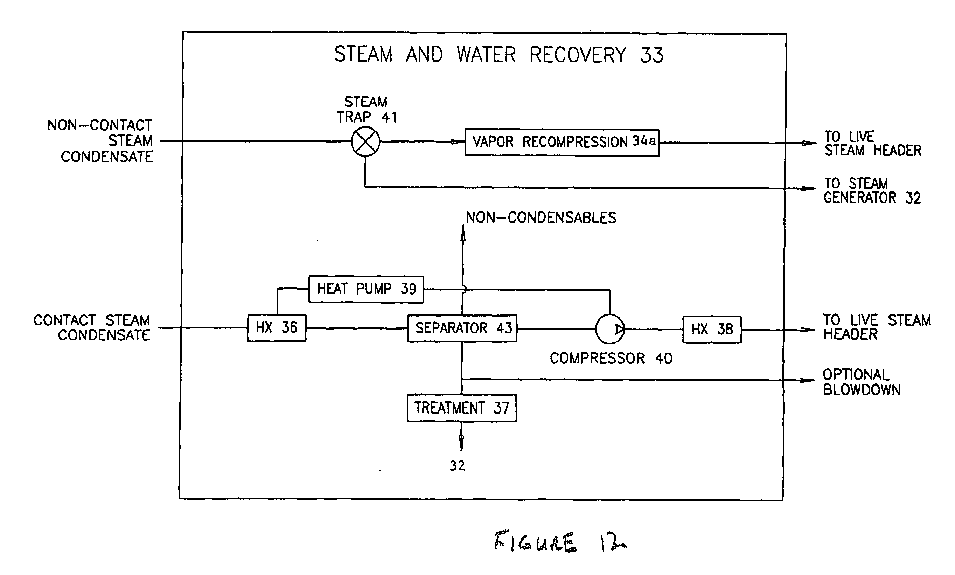

Figure 12 is a schematic for alternative process flows, in accordance with an alternative embodiment of the present invention.

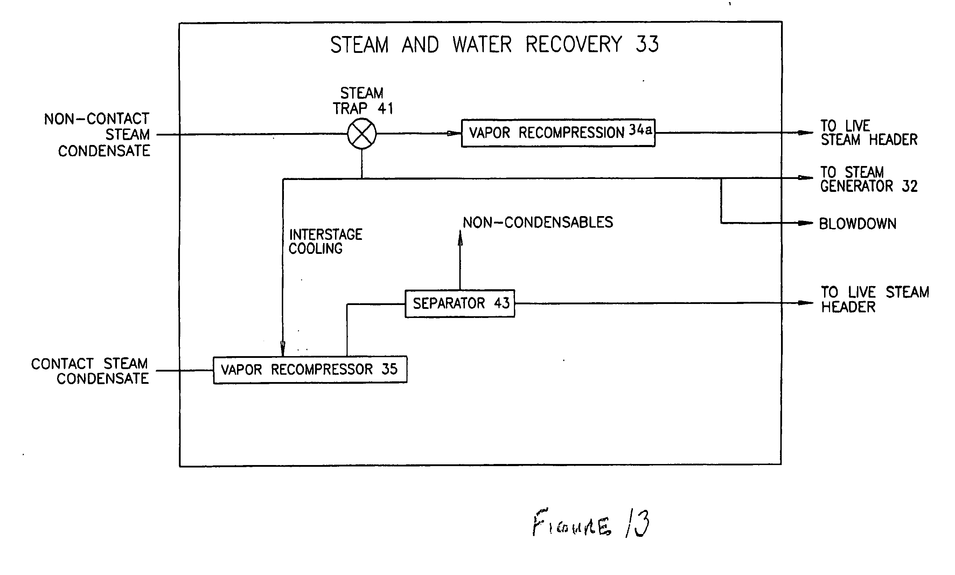

Figure 13 is a schematic for alternative process flows, in accordance with an alternative embodiment of the present invention.

### Detailed Description of the Preferred Embodiment

In accordance with the foregoing summary, the following provides a detailed description of the preferred embodiment, which is presently considered to be the best mode thereof.

In one embodiment, steam may be introduced into, and water/steam may be exhausted through a common rotating union on each end of the autoclave. The end of the autoclave may be provided with one or more manifolds to distribute the steam and collect the spent waste steam to and from the tubes in the autoclave.

In the instance where autoclave operations require autoclave material to flow through the end of the autoclave, a manifolded door must be provided. The present invention provides one or more manifolded doors, to provide a sealed manifold against a system of tubes.

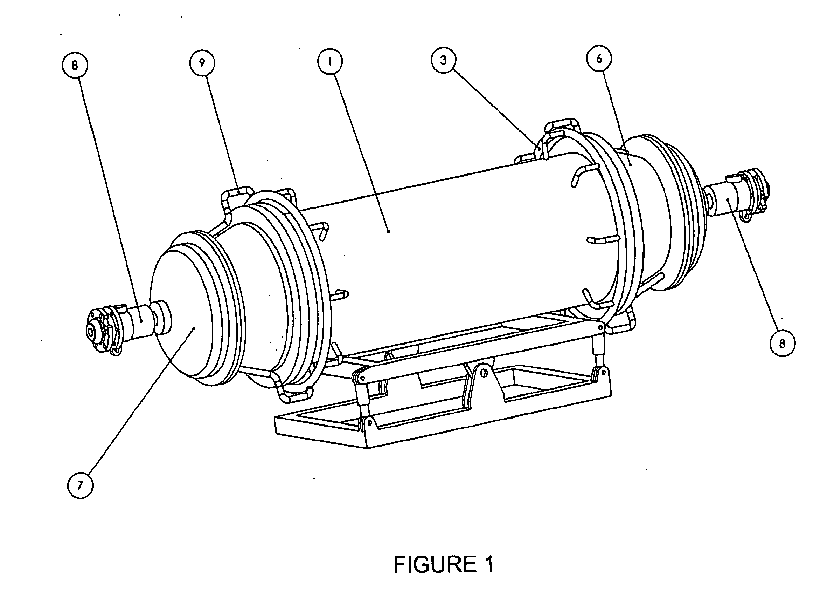

Figure 1 is a perspective view of an autoclave apparatus for processing solid waste products, in accordance with one embodiment of the present invention. Figure 1 provides a side perspective view of the autoclave 1, mounted on a fulcrum-like tilt swivel with actuators to tilt the vessel from the horizontal to be capable of leveling the load during processing, and to assist in charging the waste and discharging the treated waste product. Such actuators may be any mechanical or hydraulic arrangement or device, such as those described in the incorporated references. Figure 1 shows steam distribution plenum 3, frusto-conical vessel cone portion 6 of the autoclave vessel, hatch 7 and rotary union steam inlets 8. Hatch 7 may be fixed in the closed position for instance by action of opposing C-clamps that fit over the flange portions connecting the frusto-conical vessel cone portion 6 and the hatch 7.

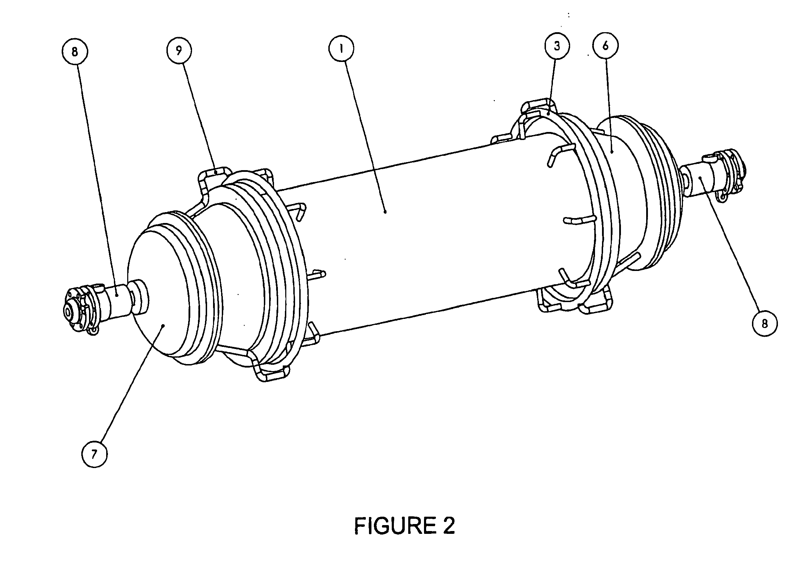

Figures 2-8 provide the autoclave vessel 1, as well as various sections through the vessel, in order to elaborate on the functions of the autoclave vessel.

Figure 2 is a perspective view of an autoclave vessel for processing solid waste products, in accordance with one embodiment of the present invention. Figure 2 shows a treatment autoclave 1 as described herein, absent the fulcrum-like tilt swivel and actuators.

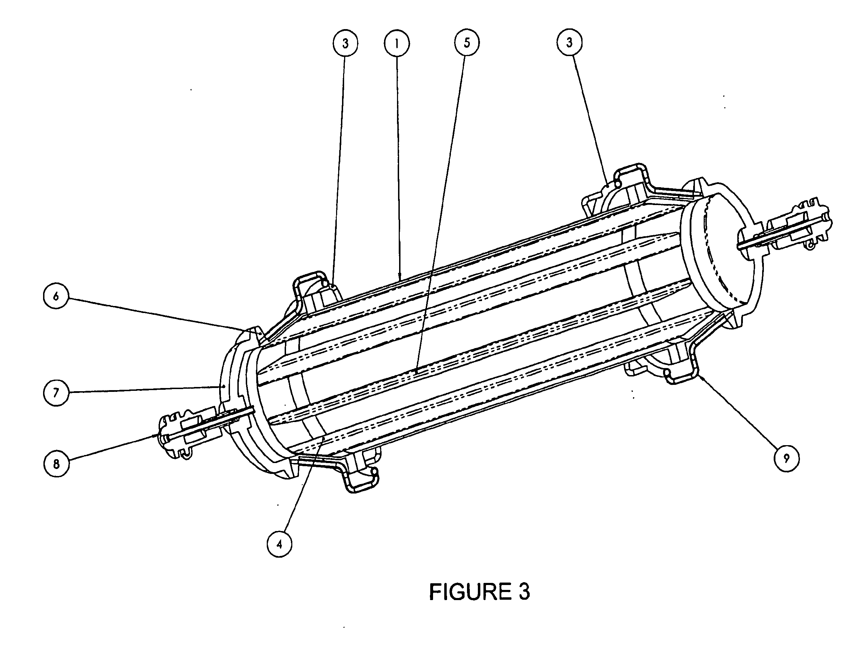

Figure 3 is a partially sectioned perspective view of an autoclave vessel for processing solid waste products, in accordance with one embodiment of the present invention. Figure 3 uses the same reference numerals as on Figures 1 and 2, and also shows a plurality of straight hollow blades 4 and 5 used in the autoclave. As can be appreciated from the cross sections, the hollow blades may be of varied cross-section and size, and preferably are straight. Figure 3 also shows frusto-conical vessel cone portion 6, hatch 7 and distributor tube 9 (which takes steam from the flange to the steam distribution plenum 3).

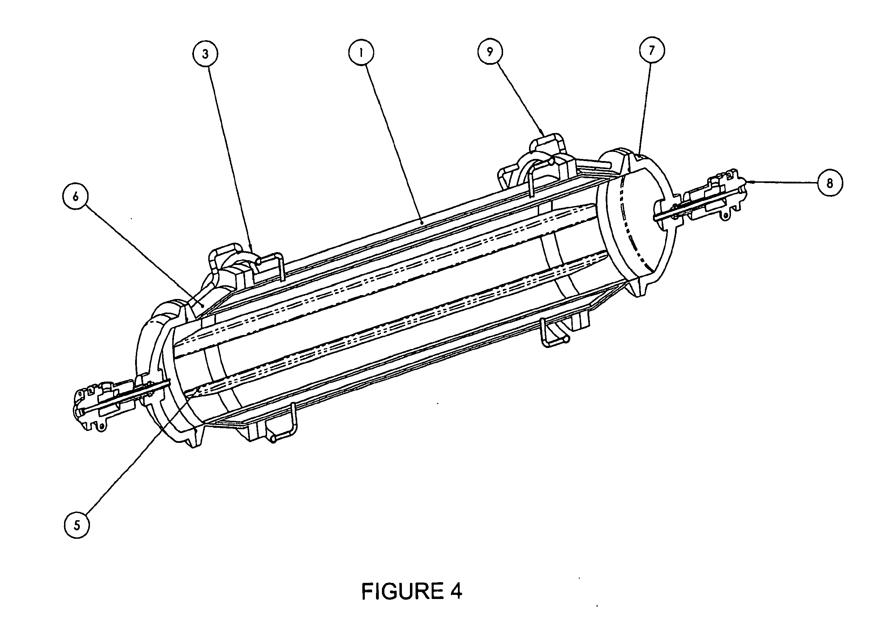

Figure 4 is a partially sectioned perspective view of an autoclave vessel for processing solid waste products, in accordance with one embodiment of the present invention, using the same reference numerals as in the aforementioned Figures.

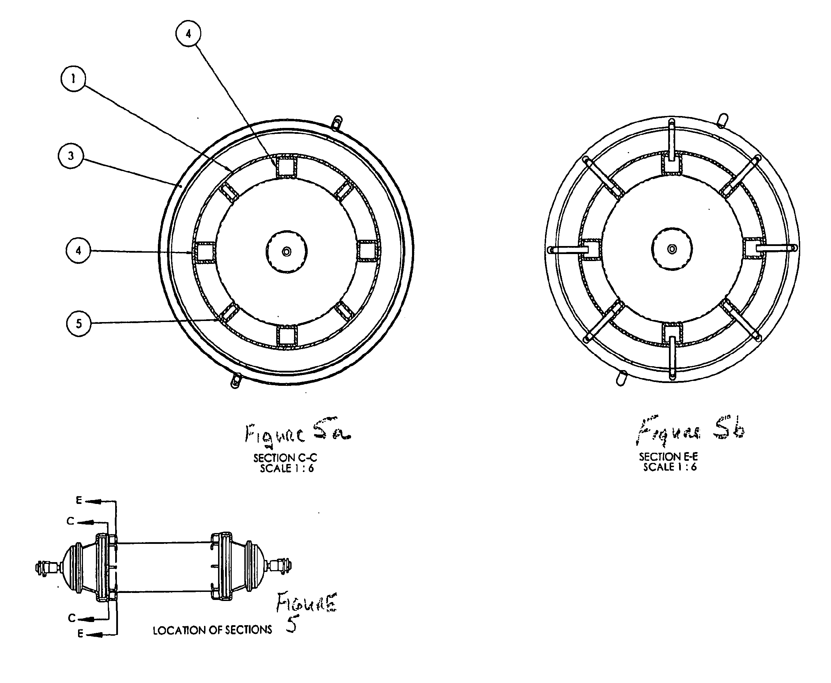

Figure 5 is an elevation view of an autoclave vessel for processing solid waste products, in accordance with one embodiment of the present invention.

Figure 5a is a longitudinal sectioned view, taken along line C-C of Figure 5, of an autoclave vessel for processing solid waste products, in accordance with one embodiment of the present invention.

Figure 5b is a longitudinal sectioned view, taken along line E-E of Figure 5, of an autoclave vessel for processing solid waste products, in accordance with one embodiment of the present invention.

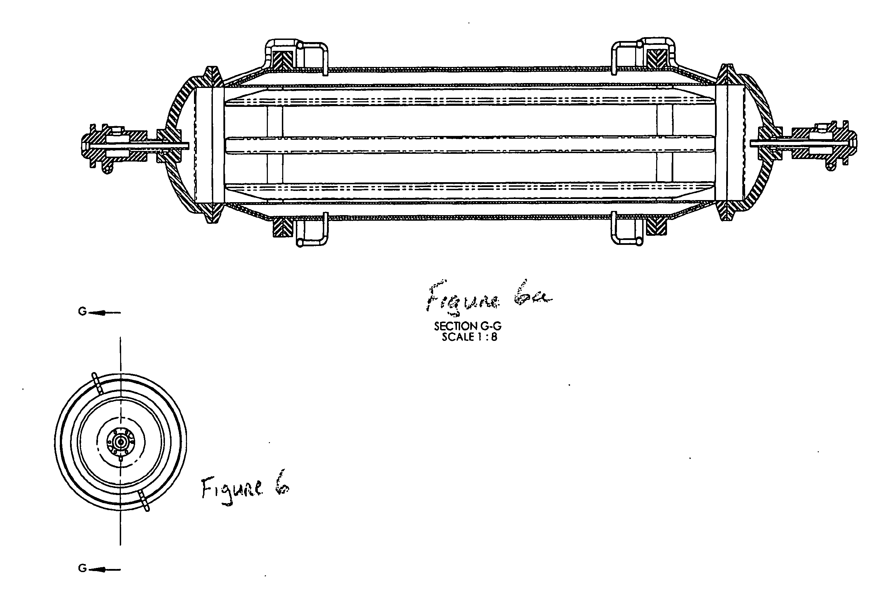

Figure 6 is a longitudinal view of an autoclave vessel for processing solid waste products, in accordance with one embodiment of the present invention.

Figure 6a is a sectioned elevation view, taken along line G-G of Figure 6, of an autoclave vessel for processing solid waste products, in accordance with one embodiment of the present invention.

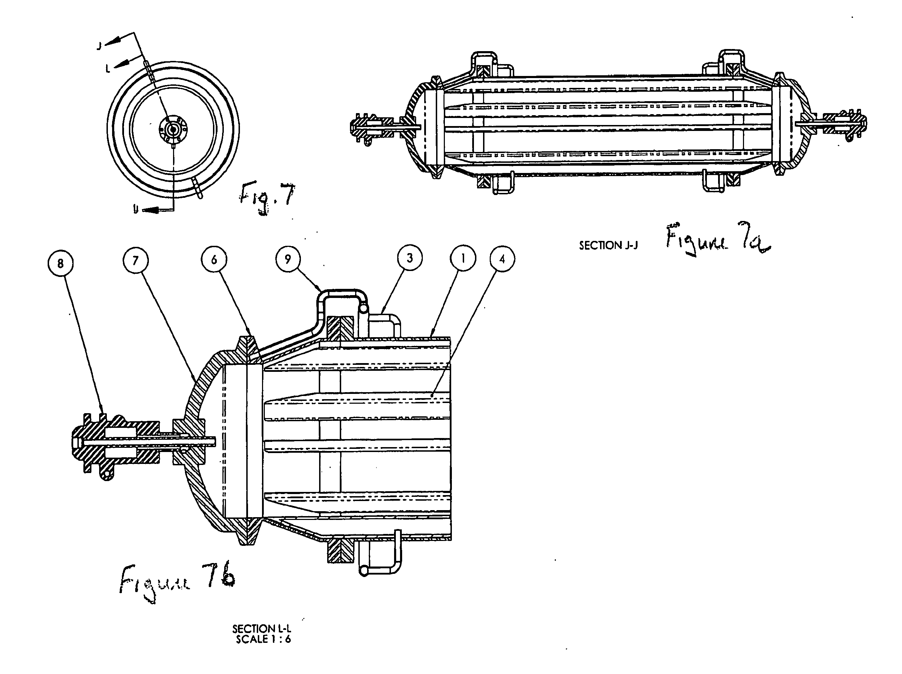

Figure 7 is a longitudinal view of an autoclave vessel for processing solid waste products, in accordance with one embodiment of the present invention.

Figure 7a is a sectioned elevation view, taken along line J-J of Figure 7, of an autoclave vessel for processing solid waste products, in accordance with one embodiment of the present invention.

Figure 7b is a detailed sectioned elevation view, taken along line L-L of Figure 7, of an autoclave vessel for processing solid waste products, in accordance with one embodiment of the present invention. These Figures use the same reference numerals as in the aforementioned Figures.

Figure 8 is sectioned perspective view of an autoclave vessel for processing solid waste products, in accordance with one embodiment of the present invention, using the same reference numerals as in the aforementioned Figures.

Figures 9 - 13 provide schematics of systems that may be provided in accordance with several embodiments of the present invention. It will be understood that the represented components may be provided in the form of commercially available components, such as heat exchangers, separators, heat recovery steam generators, gasification, combustion and power generation system, and associated steam and water conduits and valves, as well as process control systems such as systems to monitor water, steam, pressure, moisture and temperature.

Figure 9 provides a schematic for the process (steam) flow, focused on the contact steam and the autoclave. This Figure shows how the feedstock containing water is processed by the autoclave to yield contact and clean (non-contact) steam condensate respectively from the steam in contact with the waste mass, and the steam that is circulated through the hollow blades, such as 4 and 5 shown in the Figures.

The contact condensate is conducted to heat exchanger 34 which includes an air intake. This produces hot combustion gases that are in turn routed to the gasification, combustion and power generation system 31. The condensate from the heat exchanger 34 is directed to a separator, and non-condensable gases are taken therefrom and disposed of in the gasification, combustion and power generation system 31. The remaining liquid from the separator in turn is directed to a boiler water treatment system 35 where it is cleansed for reintroduction into the heat recovery steam generator (HRSG) 32. The HRSG obtains combustion gases from the gasification, combustion and power generation system 31. The HRSG 32 also generates superheated steam that may be routed to the gasification, combustion and power generation system 31. The gasification, combustion and power generation system 31 in turn produces electricity which may be used on site for any industrial purposes, such as in the water treatment system, or to operate the autoclave itself, etc. The process steam produced by the HRSG 32 may then be returned to the autoclave 30. In addition, it is preferred that the process steam be returned to the gasification, combustion and power generation system 31 for operational purposes. Superheated steam generated by the HSRG 32 may then be returned to the gasification, combustion and power generation system 31. An exhaust stack may be used to vent away excess evaporated water from the HSRG 32 as required.

The clean condensate from the non-contact steam coursed through the hollow blades may be recycled through a steam separator which feeds mechanical vapor recompression device 33 that may regenerate process steam for reuse either as contact or non-contact steam.

Clean cellulose obtained from the solid fraction of the treated waste mass may be used for any number of purposes, including for combustion in the gasification, combustion and power generation system 31.

Figure 10 provides the general schematic for the preferred embodiment of the autoclave contact steam recycling system, integrated with other functional elements of the preferred embodiment of the total autoclave system. This Figure shows the autoclave vessel 30 with conduits for contact ("contents") and non-contact ("dryer") steam, the combustion air heat exchanger 34, the separator with drain, and the conduits and valves governing the transmission of fluids to the condensed reclaimed water to the boiler and steam generator 32, as well as to the gasification, combustion and power generation system 31. Also shown is the removal and/or treatment of the non-condensables to the combustion air or to other treatment, as desired and practical. This schematic also shows that the non-contact steam may be adapted for other uses with a waste treatment facility.

Figure 11 provides a schematic for the process (steam) flow, focused on the non-contact steam flow and the autoclave, in one embodiment of the present invention. This Figure shows the autoclave vessel 30 with conduits for contact steam ("contents") to a heat exchanger 34, and for non-contact ("dryer") steam, with the non-contact steam (typically under medium pressure; 50 - 400 psi) carried to a separator device with drain, and the conduits and valves governing the transmission of fluids to the condensed reclaimed water to the boiler and steam generator 32, as well as to the mechanical vapor recompression device 33 which in turn generates reclaimed medium pressure steam that may be reused such as by being directed to the steam header in advance of the autoclave.

Figures 12 and 13 provide schematics for alternative process flows for other embodiments of the total system. Figures 12 and 13 show detailed schematics of the steam vapor and recovery system of the present invention, showing the treatment of contact and non-contact steam. Figure 12 shows the flows of contact and non-contact steam. The non-contact steam enters steam trap 41 to separate and direct condensate to steam generator 32 while the remaining steam is conducted to mechanical vapor recompression device 34a, which supplies the live steam header. The contact steam is conducted first to heat exchanger 34 which supplies heat pump 39 which in turn serves compressor 40. The condensate is sent to separator 43 which separates the steam to be supplied to compressor 40, while the condensate is sent to heat exchanger 36 and further to heat pump 39 that resupplies compressor 40 as well. The non-condensable substances may be exhausted from separator 43 as shown and may be subjected to remediation or combustion for environmental protection. The separator also provides water to water treatment unit 37 which may provide optional blowdown and HRSG 32. Separator 43 may also provide compressor 40 with reclaimed steam which is directed to heat exchanger 38 to produce steam for the live steam header.

By contrast, Figure 13 shows the flows of contact and non-contact steam. The non-contact steam enters steam trap 41 to separate and direct steam to vapor recompressor 34a, while the separated water is conducted to steam generator 32 and/or is used for blowdown and/or interstage cooling which in turn is directed to vapor recompressor 35. The contact steam is conducted first to vapor recompressor 35 which supplies separator 43 that separates the non-condensables and supplies the live steam header.

In operation of one embodiment of the present invention, the manifold system may be moved to the autoclave itself. Steam, as before, flows to the end of the autoclave 2, using a door 7, with a rotating union 8. From this rotating connector, steam is routed to a pipe 9 then to the manifold 3. The manifold distributes steam to the pipes and hollow blades within the autoclave. In this manner, the door can be opened and closed while maintaining a sealed steam supply line.

If the exit line or lines from a sealed autoclave (whether steam or water) is/are closed, and the steam is allowed to enter the autoclave, the autoclave contents will heat up, both from the contact with the hot walls of the manifolded tubes and from direct contact with the steam. Pressure in the autoclave will also increase. The temperature and pressure will rise in a dynamic and rapid manner until the autoclave and contents near the saturation temperature/pressure of the supply steam. The water content of the autoclave will also rise, as steam condenses to give up its latent heat.

Effective use of the steam tubes for drying requires that the heating steam be under a higher pressure and temperature than the autoclave contents. Three aspects to the present invention may be used to achieve this end.

In the first aspect of the present invention, one end of the autoclave provides a dual supply and exhaust system, using a commercially available two-port rotating union. Typically, one port distributes steam to a manifold as described above, and the other port receives condensate from a similarly constructed manifold.

Non-contact steam is introduced to one union into the autoclave system 30, and thence into the autoclave vanes 5, whereupon it substantially condenses, yielding its heat of condensation to the autoclave contents. The flow of the non-contact steam is regulated by valves, and the valves are adjusted such that about 15% of the introduced steam does not condense.

After passing through a set of autoclave tubes, the non-contact condensate is captured and discharged through a second rotary union.

Non-contact condensate is led to a separator tank, wherein the steam (about 15% of the total) and liquid (about 85% of the total) is separated.

The clean non-contact steam loses a little pressure through the autoclave transit: the steam is repressurized with a small mechanical vapor recompressor 33 and the regenerated steam is charged to the steam main.

The clean non-contact condensed water is returned to the steam generator 32 to create additional steam to charge to the steam main.

The process creates excess water. The steam generator (32) preferentially provides a superheat loop to generate high temperature steam from the excess water; this energy can be recovered as electrical energy with a turbine. In the preferred embodiment, the superheated steam is injected into the turbine section of a gas turbine in the power generator 31 for energy recovery.

During the cooking cycle, contact steam may be added to the autoclave contents to increase its moisture initially (assisting in the pulping process) and also providing more rapid heating. In the preferred embodiment, the added contact steam raises the autoclave moisture to the preferred pulping moisture content (in excess of 50%), and raised the autoclave interior temperature to cooking temperatures in the range of 149-173°C.

For the drying cycle, the autoclave is initially vented, releasing contact steam and lowering the pressure and temperature of the contents. The autoclave contents are held at or near ambient pressure for the remainder of the drying cycle, thus providing a lower temperature environment to receive heat from the non-contact, higher temperature, drying steam. As the contents dry, additional contact steam is generated within and released by the autoclave.

Contact steam condensate released from autoclave 30 contains a substantial quantity of latent heat. In the preferred embodiment, the contact condensate is cooled and condensed by heat exchange with heat exchanger 34. Intake air (combustion air) is heated by heat exchanger 34, thus capturing the sensible heat from the contact condensate.

Heat exchanger 34 may be a direct heat exchanger, or may be an indirect heat exchanger.

After condensation, non-condensibles are easily separated from the condensed liquid. As the non-condensibles contain volatile organics, the non-condensibles are lead to the turbine in the power generation 31 for combustion/ destruction.

Condensed liquids from the heat exchanger 34 and following separator are led to boiler water treatment 35 for cleanup and re-use.

A plurality of heating tubes may be used in order to provide a large heat transfer surface, in accordance with arrangements known in the art. Such a plurality is most effective when the autoclave contents are homogenous and of small size. In the extant application with municipal solid waste, the autoclave contents are heterogeneous and may have bulky items that render prior art unworkable for the MSW autoclave. Thus, the heating must be provided by the wall of the autoclave, and by rigid heat transfer surfaces attached to the wall.

Another invention of this disclosure utilizes non-circular tubes that conform to the interior of the rotating autoclave. In this manner, a high surface area is provided without the interferences of a complex of interior tubes. The tubes may be in the form of hollow blades in order to better distribute heat. It is also preferred that the blades be substantially straight as this best facilitates the loading and un-loading of the waste mass.

The preferred non-circular tubes are integral to the mechanical mixing processes that occur in the autoclave.

After the autoclave is loaded with the charge of waste products, the door through which the charge was loaded is shut, steam is introduced continually into the autoclave, and the autoclave becomes pressurized. Fresh steam may be continuously fed into the autoclave from the loading end, and after a predetermined processing pressure is reached, steam may be allowed to escape the autoclave into the discharge steam line.

The temperature and pressure of the autoclave are monitored, and the flow of steam is regulated to keep the process within predetermined processing ranges (typically around 7 bar, at 173°C). The autoclave is rotated at a predetermined speed (depending on the size of the autoclave). Following a cooking time interval (which is automatically controlled), the pressure in the autoclave is released, steam is used to flood the steam tubes, and the drying commences. Typical dry times of 30 minutes are experienced in 10-ton autoclaves. After drying the processed waste is removed.

A given charge of waste products may contain a wide variety of constituents, such as wood, paper, organic matter, water, etc. Each charge of waste products presents its own heat capacity and transfer profile, while there is required an overall heat absorption of the mass in order to provide an effective treatment of the waste products charge.

The information from the thermometers or thermocouples may also be used to determine the heat absorption over time as the charge of waste products heats up initially. This allows the operator (or a microprocessor or PLC controller) to extrapolate the energy needs for that charge of waste products and, based upon a comparison of the heat transfer profile, to also determine the approximate qualitative constituent make up of the charge of waste products, and thus allow the determination of the treatment time necessary to treat that particular charge.

According to the invention, steam inlets are connected to one or more saturated or superheated steam supply and to a steam receiver. In this way, the autoclave is pressurized and depressurized from one or more openings. Each opening may be provided with a pressure lock and bidirectional steam flow conduits and valves. The apparatus may also include an optional steam vacuum pump at each opening for rapid depressurizing of the autoclave. Rotating union valves may also be included to enable steam flow in- and out- of the autoclave as the autoclave is rotating or tilting. The temperature range of the autoclave contents is preferred to be in the 149-173° C temperature range during cooking. This range has been established by the applicant as providing the most rapid pulping of contents, without causing thermal degradation of plastics. Under separate application, the preferred temperature range is claimed as it provides for the inactivation of CJD "prions".

The temperature of the drying steam may be provided to be higher that the contents temperature, in order to enhance the rate of heat transfer from the blades to the autoclave contents and thereby decrease the drying time.

Alternative configurations are possible. For example, as shown in Figure 12, a heat pump 39 may be employed to condense the contact steam at heat exchanger 36. Following the removal of non-condensibles and further cleanup, the contact condensate can be compressed, and converted to live steam with the heat rejected by the heat pump at heat exchanger 38.

As another alternative, as shown in Figure 13, a mechanical vapor recompressor may be used to raise the pressure of the contact condensate vapors. In this process, the temperature of the condensate is also raised. It is desirable to provide interstage cooling in a multiple stage MVR to limit the temperature rise. About 10% by weight of water is added to the MVR for cooling, and may be extracted from steam trap 41.

Figure 13 also shows a bleed for non-condensables. Here, a thermostatic trap is used to preferentially remove non-condensables such as air and volatile organics.

## Claims

1. An apparatus for processing solid waste products, said apparatus comprising:
(a) a rotatably mounted cylindrical autoclave (1) having an interior surface and two ends, at least one end terminating in a hatch (7) that may be opened to allow access to the interior of said autoclave (1) and sealably closed to allow pressurization of said autoclave (1);
(b) an autoclave steam inlet (8) for injecting contact steam into said autoclave (1) so as to come into contact with waste material placed into said autoclave (1)
**characterised by**
(c) a plurality of straight hollow blades adapted to conduct non-contact steam and protruding from said interior surface of said autoclave (1);
(d) at least one blade steam inlet for injecting steam into said blades,
the blades extending from one end of the autoclave (1) to the other.

2. An apparatus according to claim 1 wherein said autoclave (1) additionally comprises a waste steam outlet adapted to conduct waste contact steam from said interior of said autoclave (1) and wherein another waste steam outlet is adapted to conduct said non-contact waste steam from said blades.

3. An apparatus according to claim 2 wherein said autoclave steam inlet (8) and said blade steam inlet are each provided with a rotating connector so as to permit the supply of steam therethrough while said autoclave (1) is rotated.

4. An apparatus according to claim 2 wherein additionally comprising a variable flow valve used to dynamically control the exit steam quality.

5. An apparatus according to claim 1 wherein (1) said autoclave (1) additionally comprises a waste steam outlet adapted to conduct waste steam from said interior of said autoclave (1) and wherein another waste steam outlet is adapted to conduct non-contact waste steam from said blades and (2) a device is adapted to reconstitute said waste steam from said hollow blades and to reintroduce reconstituted steam into said autoclave steam inlet (8) and or the inlets of other autoclaves.

6. An apparatus according to claim 1 additionally comprising a steam separator followed by mechanical vapor recompression device for separated steam and a boiler/steam generator for separated water.

7. An apparatus according to claim 1 wherein said autoclave (1) is adapted to be tilted at an angle from the horizontal so as to cause the liquid condensate within said blades to migrate from the steam supply end to the steam exhaust end.

8. An apparatus according to claim 7, additionally comprising means for controlling said autoclave (1) tilt by sensing the blade steam exhaust condensate conditions and adjusting the tilt to regulate the production and exhaust of condensate in said blades.

9. A method of processing solid waste material in an autoclave (1), comprising:
(1) loading the autoclave (1) with a charge of solid waste products, said autoclave (1) comprising:
(a) a rotatably mounted cylindrical autoclave (1) having an interior surface and two ends, at least one end terminating in a hatch (7) that may be opened to allow access to the interior of said autoclave (1) and sealably closed to allow pressurization of said autoclave (1);
(b) an autoclave steam inlet (8) for injecting steam into said autoclave (1) so as to come into contact with waste material placed into said autoclave (1);
(c) a plurality of straight hollow blades adapted to conduct steam and protruding from said interior surface of said autoclave (1); and
(d) at least one blade steam inlet for injecting steam into said blades, a first waste stream outlet for conducting waste steam from the interior of the autoclave (1) and a second waste stream outlet for conducting non-contact waste steam from the blades;
(2) sealing the autoclave (1);
(3) rotating said autoclave (1) so as to cause said charge of solid waste material to be moved from the bottom of said autoclave (1) to toward the top of said autoclave (1) while introducing contact steam into said autoclave (1), so as to produce sterile processed waste;; and
(5) thereafter depressurizing the autoclave (1) and unloading the processed waste therefrom,
**characterised in that** the blades extend from one end of the autoclave (1) to the other.

10. A method according to claim 9, wherein the plurality of hollow blades are straight hollow blades and the steam is introduced into the blades so as to produce sterile dry processed waste.

11. A method according to claim 10, wherein said steam introduced into said blades is substantially comprised of reconstituted non-contact waste steam obtained from said autoclave (1).

12. A method according to claim 11, wherein said steam introduced into said blades comprises reconstituted non-contact waste steam obtained from said autoclave (1), and additionally comprising a variable flow valve adapted to control the flow of said waste steam.

13. A method according to claim 10, wherein said device adapted to reconstitute said non-contact waste steam is a steam separator followed by mechanical vapor recompression device for separated steam and a boiler/steam generator for separated water, wherein the thermal energy of said contact condensate is recovered by heat transfer to combustion air, to steam generation, and/or by direct mechanical vapor recompression.

## Patentansprüche

1. Vorrichtung zum Verarbeiten von festen Abfallprodukten, wobei die genannte Vorrichtung Folgendes umfasst:
(a) einen drehbar montierten zylindrischen Autoklav (1) mit einer Innenfläche und zwei Enden, wobei wenigstens ein Ende in einer Luke (7) endet, die geöffnet werden kann, um Zugang zum Innern des genannten Autoklavs (1) zu erhalten, und die dichtend verschlossen werden kann, damit der genannte Autoklav (1) unter Druck gesetzt werden kann;
(b) einen Autoklavdampfeinlass (8) zum Injizieren von Kontaktdampf in den genannten Autoklav (1), so dass er mit Abfallmaterial in dem genannten Autoklav (1) in Kontakt kommt,
**gekennzeichnet durch**
(c) mehrere gerade hohle Lamellen zum Leiten von Nicht-Kontakt-Dampf, die von der genannten Innenfläche des genannten Autoklavs (1) vorstehen;
(d) wenigstens einen Lamellendampfeinlass zum Injizieren von Dampf in die genannten Lamellen, wobei die Lamellen von einem Ende des Autoklavs (1) zum anderen verlaufen.

2. Vorrichtung nach Anspruch 1, wobei der genannte Autoklav (1) ferner einen Abdampfauslass zum Leiten von Kontaktabdampf aus dem genannten Innern des genannten Autoklavs (1) umfasst und wobei der andere Abdampfauslass zum Leiten des genannten Nicht-Kontakt-Abdampfes von den genannten Lamellen ausgelegt ist.

3. Vorrichtung nach Anspruch 2, wobei der genannte Autoklavdampfeinlass (8) und der genannte Lamellendampfeinlass jeweils mit einem drehbaren Anschluss versehen sind, durch den Dampf zugeführt werden kann, während der genannte Autoklav (1) gedreht wird.

4. Vorrichtung nach Anspruch 2, die zusätzlich ein variables Strömungsventil zum dynamischen Regeln der Qualität des austretenden Dampfes umfasst.

5. Vorrichtung nach Anspruch 1, wobei (1) der genannte Autoklav (1) zusätzlich einen Abdampfauslass zum Leiten von Abdampf von dem genannten Innern des genannten Autoklavs (1) umfasst und wobei ein anderer Abdampfauslass zum Leiten von Nicht-Kontakt-Abdampf von den genannten Lamellen ausgelegt ist, und (2) eine Vorrichtung zum Rekonstituieren des genannten Abdampfs von den genannten hohlen Lamellen und zum Wiedereinleiten von rekonstituiertem Dampf in den genannten Autoklavdampfeinlass (8) und/oder in die Einlässe von anderen Autoklaven ausgelegt ist.

6. Vorrichtung nach Anspruch 1, die zusätzlich einen Dampfabscheider gefolgt von einer Vorrichtung zur mechanischen Dampfrekompression für abgeschiedenen Dampf und einen Kessel/Dampfgenerator für abgeschiedenes Wasser umfasst.

7. Vorrichtung nach Anspruch 1, wobei der genannte Autoklav (1) zum Kippen in einem Winkel von der Horizontalen ausgelegt ist, um zu bewirken, dass das flüssige Kondensat in den genannten Lamellen vom Dampfeinleitungsende zum Dampfauslassende wandert.

8. Vorrichtung nach Anspruch 7, die zusätzlich Mittel zum Steuern des Kippens des genannten Autoklavs (1) durch Erfassen des Zustands des Lamellendampfauslasskondensats und zum Justieren des Kippens zum Regulieren von Produktion und Auslass von Kondensat in den genannten Lamellen umfasst.

9. Verfahren zum Verarbeiten von festem Abfallmaterial in einem Autoklav (1), das Folgendes beinhaltet:
(1) Beladen des Autoklavs (1) mit einer Charge von festen Abfallprodukten, wobei der genannte Autoklav (1) Folgendes umfasst:
(a) einen drehbar montierten zylindrischen Autoklav (1) mit einer Innenfläche und zwei Enden, wobei wenigstens ein Ende in einer Luke (7) endet, die geöffnet werden kann, um Zugang zum Innern des genannten Autoklavs (1) zu erhalten, und die dichtend verschlossen werden kann, damit der genannte Autoklav (1) unter Druck gesetzt werden kann;
(b) einen Autoklavdampfeinlass (8) zum Injizieren von Kontaktdampf in den genannten Autoklav (1), so dass er mit Abfallmaterial in dem genannten Autoklav (1) in Kontakt kommt,
(c) mehrere gerade hohle Lamellen zum Leiten von Nicht-Kontakt-Dampf, die von der genannten Innenfläche des genannten Autoklavs (1) vorstehen; und
(d) wenigstens einen Lamellendampfeinlass zum Injizieren von Dampf in die genannten Lamellen, einen ersten Abdampfauslass zum Leiten von Abdampf aus dem Innern des Autoklavs (1) und einen zweiten Abdampfauslass zum Leiten von Nicht-Kontakt-Abdampf aus den Lamellen,
(2) Verschließen des Autoklavs (1);
(3) Drehen des genannten Autoklavs (1), um eine Bewegung der genannten Charge von festem Abfallmaterial vom Boden des genannten Autoklavs (1) zum oberen Teil des genannten Autoklavs (1) zu bewirken, während Kontaktdampf in den genannten Autoklav (1) geleitet wird, um sterilen verarbeiteten Abfall zu erzeugen; und
(5) danach Ablassen des Drucks aus dem Autoklav (1) und Herausnehmen des verarbeiteten Abfalls daraus,
**dadurch gekennzeichnet, dass** die Lamellen von einem Ende des Autoklavs (1) zum anderen verlaufen.

10. Verfahren nach Anspruch 9, wobei die mehreren hohlen Lamellen gerade hohle Lamellen sind und der Dampf so in die Lamellen eingeleitet wird, dass steriler trockener verarbeiteter Abfall erzeugt wird.

11. Verfahren nach Anspruch 10, wobei der genannte in die genannten Lamellen eingeleitete Dampf im Wesentlichen aus rekonstituiertem Nicht-Kontakt-Abdampf besteht, der von dem genannten Autoklav (1) erhalten wurde.

12. Verfahren nach Anspruch 11, wobei der genannte in die genannten Lamellen eingeleitete Dampf rekonstituierten Nicht-Kontakt-Abdampf umfasst, der von dem genannten Autoklav (1) erhalten wurde, und zusätzlich ein variables Strömungsventil zum Regeln des Flusses des genannten Abdampfes umfasst.

13. Verfahren nach Anspruch 10, wobei die genannte Vorrichtung zum Rekonstituieren des genannten Nicht-Kontakt-Abdampfes ein Dampfabscheider gefolgt von einer Vorrichtung zur mechanischen Dampfrekompression für abgeschiedenen Dampf und einem Kessel/Dampfgenerator für abgeschiedenes Wasser ist, wobei die Wärmeenergie des genannten Kontaktkondensats durch Wärmeübertragung auf Brennluft, zur Dampferzeugung und/oder durch direkte mechanische Dampfrekompression zurückgewonnen wird.

## Revendications

1. Appareil de traitement de produits de déchets solides, ledit appareil comprenant :
(a) un autoclave cylindrique monté avec faculté de rotation (1) ayant une surface intérieure et deux extrémités, au moins une extrémité se terminant dans une trappe (7) qui peut être ouverte pour pouvoir accéder à l'intérieur dudit autoclave (1) et fermée de manière étanche pour pouvoir pressuriser ledit autoclave (1) ;
(b) une admission de vapeur d'autoclave (8) pour injecter une vapeur de contact dans ledit autoclave (1) de façon à ce qu'elle entre en contact avec une substance de déchet placée dans ledit autoclave (1),
**caractérisé par**
(c) une pluralité de pales creuses droites adaptées pour acheminer une vapeur non de contact et saillant de ladite surface intérieure dudit autoclave (1) ;
(d) au moins une admission de vapeur de pales pour injecter de la vapeur dans lesdites pales,
les pales s'étendant depuis une extrémité de l'autoclave (1) jusqu'à l'autre.

2. Appareil selon la revendication 1, dans lequel ledit autoclave (1) comprend de plus une sortie de vapeur de déchet adaptée pour acheminer une vapeur de contact de déchet depuis ledit intérieur dudit autoclave (1) et dans lequel une autre sortie de vapeur de déchet est adaptée pour acheminer ladite vapeur de déchet non de contact depuis lesdites pales.

3. Appareil selon la revendication 2, dans lequel ladite admission de vapeur d'autoclave (8) et ladite admission de vapeur de pale sont chacune dotées d'un connecteur rotatif de manière à permettre l'alimentation de vapeur à travers celui-ci quand ledit autoclave (1) est en rotation.

4. Appareil selon la revendication 2, comprenant de plus un clapet d'écoulement variable utilisé pour commander dynamiquement la qualité de la vapeur de sortie.

5. Appareil selon la revendication 2, dans lequel ledit autoclave (1) comprend de plus une sortie de vapeur de déchet adaptée pour acheminer une vapeur de déchet depuis ledit intérieur dudit autoclave (1) et dans lequel une autre sortie de vapeur de déchet est adaptée pour acheminer une vapeur de déchet sans contact depuis lesdites pales et (2) un dispositif est adapté pour reconstituer ladite vapeur de déchet depuis lesdites pales creuses et réintroduire la vapeur reconstituée dans ladite admission de vapeur d'autoclave (8) et ou les admissions d'autres autoclaves.

6. Appareil selon la revendication 2, comprenant de plus un séparateur de vapeur suivi d'un dispositif mécanique de recompression de vapeur pour la vapeur séparée et une chaudière/un générateur de vapeur pour l'eau séparée.

7. Appareil selon la revendication 1, dans lequel ledit autoclave (1) est adapté pour être incliné à un angle par rapport à l'horizontale de manière à ce que le condensat liquide dans lesdites pales migre de l'extrémité d'alimentation de vapeur à l'extrémité d'échappement de vapeur.

8. Appareil selon la revendication 7, comprenant de plus un moyen pour commander l'inclinaison dudit autoclave (1) en détectant les conditions du condensat d'échappement de vapeur de pales et réglant l'inclinaison pour réguler la production et l'échappement du condensat dans lesdites pales.

9. Procédé de traitement d'un substance de déchet solide dans un autoclave (1), comprenant :
(1) le chargement de l'autoclave (1) avec une charge de produits de déchets solides, ledit autoclave (1) comprenant :
(a) un autoclave cylindrique monté avec faculté de rotation (1) ayant une surface intérieure et deux extrémités, au moins une extrémité se terminant dans une trappe (7) qui peut être ouverte pour pouvoir accéder à l'intérieur dudit autoclave (1) et fermée de manière étanche pour pouvoir pressuriser ledit autoclave (1) ;
(b) une admission de vapeur d'autoclave (8) pour injecter une vapeur dans ledit autoclave (1) de façon à ce qu'elle entre en contact avec une substance de déchet placée dans ledit autoclave (1),
(c) une pluralité de pales creuses droites adaptées pour acheminer une vapeur non de contact et saillant de ladite surface intérieure dudit autoclave (1) ; et
(d) au moins une admission de vapeur de pales pour injecter de la vapeur dans lesdites pales, une première sortie de vapeur de déchet pour acheminer la vapeur de déchet depuis l'intérieur de l'autoclave (1) et une seconde sortie de vapeur de déchet pour acheminer la vapeur de déchet sans contact depuis les pales ;
(2) la fermeture de l'autoclave (1) ;
(3) la rotation dudit autoclave (1) de manière à déplacer ladite charge de substance de déchet solide du fond dudit autoclave (1) vers le haut dudit autoclave (1) tout en introduisant la vapeur de contact dans ledit autoclave (1), de manière à produire un déchet traité stérile ; et
(5) la dépressurisation consécutive de l'autoclave (1) et le déchargement du déchet traité hors de celui-ci,
**caractérisé en ce que** les pales s'étendent d'une extrémité de l'autoclave (1) à l'autre.

10. Procédé selon la revendication 9, dans lequel la pluralité de pales creuses consiste en pales creuses droites et la vapeur est introduite dans les pales de manière à produire un déchet traité sec stérile.

11. Procédé selon la revendication 10, dans lequel ladite vapeur introduite dans lesdites pales consiste sensiblement en une vapeur de déchet sans contact reconstitué provenant dudit autoclave (1).

12. Procédé selon la revendication 11, dans lequel ladite vapeur introduite dans lesdites pales consiste en vapeur de déchet sans contact reconstituée provenant dudit autoclave (1) et comprenant de plus un clapet d'écoulement variable adapté pour commander l'écoulement de ladite vapeur de déchet.

13. Procédé selon la revendication 10, dans lequel ledit dispositif adapté pour reconstituer ladite vapeur de déchet sans contact est un séparateur de vapeur suivi d'un dispositif de recompression de vapeur mécanique pour la vapeur séparée et d'une chaudière/d'un générateur de vapeur pour l'eau séparée, dans lequel l'énergie thermique dudit condensat de contact est récupérée par transfert de chaleur en air de combustion, en génération de vapeur et/ou par recompression mécanique directe de la vapeur.
